# EUROPEAN PATENT APPLICATION

(11) **EP 4 803 605 A1**
(43) Date of publication of application: **09.09.2026**
(21) Application number: 25382214.2
(22) Date of filing: 07.03.2025
(51) Int. Cl.: C12M 1/107, C12M 3/00, C12M 1/36

(54) **METHOD OF PRODUCING BIOGAS**

(71) Applicant: Verdalia Bioenergy Spain, S.L., 28006 Madrid (ES)
(72) Inventor: The designation of the inventor has not yet been filed
(74) Representative: V.O.

(57) **Abstract**

Method of producing biogas from agro-industrial feed using a biogas production arrangement, wherein the biogas production arrangement comprises:
- a group of biogas digesters, wherein each biogas digester of the group of biogas digesters comprises:
- a local controller;
- operational components operatively connected to the local controller and arranged to manipulate biogas production in the corresponding biogas digester based on instructions provided by the local controller; and
- a sensor arranged to measure at least one process parameter, said sensor operatively connected to the local controller.

## Description

### TECHNICAL FIELD

Various aspects and examples thereof relate to the field of biogas digesters and the production of biogas, such as biomethane.

### BACKGROUND

Biogas is typically produced in a process comprising the degradation of organic waste such as manure, food scraps and agricultural products in an oxygen-free environment through anaerobic digestion using, for example, a biogas digester. During break down of the organic waste, a mixture of gases is produced that comprises methane and carbon dioxide. Biogas is considered a renewable energy source. The organic waste used to produce biogas may originate from previously absorbed carbon dioxide by the organic waste, e.g. carbon dioxide absorbed by plants before the plants are broken down through anaerobic digestion.

Biogas production is a complex process, in particular when an attempt is made to optimize production of biogas. These challenges may arise from an inconsistent feedstock supply as changes in organic waste quantity and quality affect gas yield. Furthermore, maintaining a stable process is important as fluctuations in for example pH, ammonia buildup and volatile fatty acid accumulation may negatively affect the production of biogas, and may be damaging to the methane-producing microbes present in the biogas digester in the long term.

Optimizing biogas production and maintaining a stable process may be challenging, as the anaerobic digestion taking place in the biogas digester relies on delicate microbial interactions that are sensitive to environmental and operational changes. For example, temperature variations may affect microbial activity when the temperature is too high or too low. It may be difficult to operate the biogas digester such that a stable process is maintained, as it may not be known before starting the process what parameters correspond to a stable process. Therefore, optimizing biogas production may be considered an even more difficult challenge. Commonly, a trial-and-error approach is used to optimize biogas production. Finding the process parameters may take a long time to find, as it depends on a large number of variables. Furthermore, not all process parameters may be controlled such as the outside temperature or the available feed. This challenge is further compounded by the fact that the effects of changing some parameters may result in changes to the process over a relatively long-time window, e.g. hours or days.

### SUMMARY

It is preferred to provide for method of producing biogas from agro-industrial feed that may be easily optimized and maintained, e.g. by having a stable biogas production process. In particular, it is preferred to provide for a method of producing biogas that reduces, and possibility eliminates, the trial-and-error process when operating and optimizing the biogas production process such that a reliable and cost-efficient source of biogas may be provided.

A first aspect provides a method of producing biogas from agro-industrial feed using a biogas production arrangement. The biogas production arrangement comprises a group of biogas digesters. In the context of the invention, a group of biogas digesters comprises a number of biogas digesters that can be indicated with a positive integer, e.g. 1, 2, 3 or 4. Each biogas digester of the group of biogas digesters comprises a local controller, operational components operatively connected to the local controller and arranged to manipulate biogas production in the corresponding biogas digester based on instructions provided by the local controller. Each biogas digester of the group of biogas digesters comprises a sensor arranged to measure at least one process parameter. The sensor is operatively connected to the local controller of the corresponding biogas digester.

The biogas production arrangement further comprises a central controller operatively connected to each local controller of the group of biogas digesters and a data storage operatively connected to the central controller. The data storage comprises historical process parameter data. The method of producing biogas comprises the steps of:
- measuring the at least one process parameter of the biogas digester using each sensor of the group of biogas digesters, thereby generating measured process parameter data;
- transmitting to the central controller, by each local controller of the group of biogas digesters, the measured process parameter data;
- determining, based on the measured process parameter data and on the historical process parameter data, the instructions for the operational components of at least one biogas digester of the group of biogas digesters, using an algorithmic model provided on the central controller;
- attempt to transmit, by the central controller to the local controller of the at least one biogas digester, the instructions for the operational components of the at least one biogas digester; and, if the attempt to transmit was successful,
- instructing the operational components of the at least one biogas digester, using the local controller, such that the biogas production of the at least one biogas digester is adjusted.

Advantageously, using the method of production biogas from agro-industrial feed, it is possible to optimize biogas production from a group of biogas digesters using historical process parameter data in combination with data of at least one process parameter. Optimizing biogas production may, for example, be maximizing biogas output based on the feedstock in the biogas digester, producing a biogas output matching a certain demand or minimizing electricity consumption of the biogas production arrangement while maintaining a certain biogas production throughput.

Historical process parameter data comprises information may comprise information about a previous state of the biogas digester, e.g. contents of the feedstock, age of the microbiological organisms, temperature inside the digester and / or the position of the operational components and relate this to a certain production of biogas of the corresponding biogas digester. Thus, from the historical process parameter data a biogas production rate may be determined that is preferred, i.e. the optimized biogas production.

By measuring the current, or actual, state of the same parameters comprised by the historical process parameter data, thereby generating the measured process parameter data, it is possible to determine the difference between the current state of the biogas digester compared to the state of the biogas digester when an optimized amount of biogas was produced in the past. As a result, it is possible to determine a configuration of the operational components that may result in the desired biogas production, without over- or undershooting the target as may happen with common biogas production processes. By replacing the commonly used trial-and-error approach for optimizing biogas production with the disclosed method, the optimized biogas production may also more quickly be obtained resulting in an overall more efficient operation of the biogas production arrangement.

In a further advantage, complexity of production of biogas production from agro-industrial feed is greatly reduced as not all the operational components need to be controlled individually by an operator, as simply inserting the required amount of biogas production may be sufficient to determine to correct configuration of operational components.

In the context of the invention, agro-industrial feed may be understood as organic waste and / or by-products formed from agricultural and food-processing industries that may be used as feed for biodigesters. Agro-industrial feed may comprise a high level of biodegradable organic matter, such that it may be particularly suitable for anaerobic digestion to produce biogas. Possible sources of agro-industrial feed are fruit and vegetable waste, animal manure, milk processing waste, coffee pulp and starch residues. Agro-industrial feed may be pre-processed before being fed to a biodigester, e.g. by shredding and / or heating. Agro-industrial feed may be a mix of different organic waste and / or by-products, from different kinds of industry.

The biogas digester is arranged to break down agro-industrial feed in an oxygen-free environment, such as a digestion chamber provided in the biogas digester, through anaerobic digestion. During anaerobic digestion biogas is produced, which comprises methane gas, and digestate. Digestate may be used as a fertilizer.

The sensor of a biogas digester of the group of biogas digesters may be any kind of sensor arranged to measure the at least one process parameter. For example, if the at least one process parameter is the temperature inside the digestion chamber, a thermometer may be provided in the digestion chamber. If more than a single process parameter is measured, the sensor may comprise various sub-sensors such that each sub-sensor is arranged to measure a corresponding process parameter. For example, if the process parameters are temperature on the outside of the biogas digester and the pH value on the inside of the biogas digester, e.g. in the digestion chamber, a thermometer and a pH meter may be provided as a sub-sensors such that the sensor may measure both the process parameters. In order to improve measurement accuracy, multiple sensors and / or sub-sensors may be provided measuring the same process parameter. From this, the process parameter may be determined as an average of sub-process parameters, each measured by a corresponding (sub)sensor.

The operative connection between the operational components and the local controller, the sensor and local controller, the local controller and central controller and / or the central controller and the data storage may be a physical connection, e.g. a wired connection, or a wireless connection. Such a wireless connection may be, depending on the range between the various components and the required amount of data transmission, Bluetooth based, WiFi based, RFID based and / or internet based.

In an implementation of the method of producing biogas, the group of biogas digesters comprises a plurality of biogas digesters. Advantageously, when the biogas production arrangement comprises plurality of biogas digesters, more data may be collected and the instructions provided by the algorithmic model on the central controller may result in the expected biogas production rate. Each of the biogas digesters comprises its own sensor arranged to measure at least one process parameter that, once measured, forms the measured process parameter of the corresponding biogas digester. The measured process parameter of each biogas digester is centrally processed using an algorithmic model on a central controller.

In an implementation of the method of producing biogas, the method further comprises the step of:
- appending, by the central controller, the measured process parameter data to the historical process parameter data. Advantageously, by appending, or adding, the measured process parameter data to the historical process parameter data, a larger dataset of historical process parameter data may be developed. A larger dataset may account for even more different configurations, such that the production of biogas may be even more accurately controlled. A large dataset of historical process parameter data may be in particular advantageous when accounting for parameters that may not be controlled by the biogas production arrangement, such as the outside temperature. For example, the outside temperature may influence the temperature on the inside of the biogas digester, which may influence the production of biogas. Thus, in order to have the same biogas production at different outside temperatures, different settings of the operational configurations may be needed. A large historical process parameter data set, comprising many different process parameters that may or may not be individually controllable or manipulated by the operational components, may thus account for a wide range of possible settings such that an optimized biogas production rate may achieved quickly.

In another implementation of the method, the process parameter comprises at least one of a operational process parameter indicative of the operational performance of the corresponding biogas digester and a microbial population process parameter indicative of the health of the microbial population in the corresponding biogas digester. The historical data comprises historical operational data and historical microbial population data. The components that may be indicative of the operational performance may relate to the inputs of the biogas digester, e.g. the electricity required to operate a biogas digester, the composition of the agro-industrial feed used to produce the biogas, and the outputs of the biogas digester, e.g. the amount of biogas produced. These parameters may be relatively convenient to determine, while having a significant impact on the overall performance of the biogas production arrangement.

Thus, by measuring operational process parameters the algorithmic model on the central controller may provide relatively accurate results. Additionally or alternatively, microbial population process parameter data may be gathered and monitored in order to control ensure that the health of the microbial population. Ensuring the health of the microbial population may be important to maintain a constant output of biogas production as an unhealthy, or dying, microbial population, may lessen the future biogas output of a digester.

In a further implementation of the method, the operational process parameter comprises at least one of biogas production rate data, temperature data, ammonia concentration data, pH level data and microbial activity data. The operational process parameter is preferably measured in real time. The mentioned operational process parameter may further enhance the accuracy of the results from the algorithmic model. Real time measurements may be advantageous to maintain steady monitoring of the biogas digester, such that upsets may be timely noticed and corrective measures may be taken. For example, a sudden and unexpected drop in biogas production rate may be indicative of an upset in the biogas digester and, if all other measured data seems relatively normal, may be an indicator that an operator should inspect the digester for any malfunctions.

In yet a further implementation of the method, the microbial population process parameter comprises at least one of microbial function data, microbial diversity data, microbial dynamics data and microbial species data. The microbial population process parameters may advantageously be used to develop and maintain a healthy and resistant microbial population inside a biogas digester over a relatively long time frame, e.g. hours, days or weeks.

Microbial function data may be indicative for the metabolic activities of the microbial groups that are involved in the anaerobic digestion taking place in the biogas digester during use. These microbial groups may comprise hydrolytic bacteria that may break down complex organic matter, acidogenic bacteria that may produce volatile fatty acids, acetogenic bacteria, that may convert acids into acetate, hydrogen and carbon dioxide, and methanogenic archaea that produce methane. Data relating to the metabolic activities of these microbial groups may be used by the algorithmic model on the central controller to optimize biogas production.

Microbial diversity data may be used by the algorithmic model to determine the variety of different microbial species present in the biogas digester. A large variety of microbial species may ensure a stable digestation process and may allow for breakdown of a wider range of organic materials. A high microbial diversity may improve the resilience against fluctuations in temperature, pH and reducing risk of process failures.

Microbial dynamics data may be used by the algorithmic model on the central controller to track the shift in populations of microbial species and the interaction of said species over time. Changes in feed, e.g. in agro-industrial feed provided to the biogas digester, temperature variations and pH fluctuations may influence the microbial composition and activity. Tracking the microbial dynamics data, and by adjusting the instructions for the operational components of a corresponding biogas digester accordingly, a balanced microbial ecosystem may be provided and negative processes such as acidification and ammonia inhibition may be reduced or prevented.

Microbial species data may allow the algorithmic model to identify the specific bacteria and / or archaea that may be involved in biogas production in the corresponding biogas digester. By knowing the dominant species in the corresponding biogas digester, may help in providing strategies to optimize biogas production, for example by adding microbial inoculants and / or adjusting environmental conditions for optimal microbial growth.

In yet a further implementation of the method, the data storage further comprises external data, said external data comprising at least one of weather data, georeferenced data, agro-industrial feed availability, land availability for digestation and energy scarcity data. The instructions for the operational components for each corresponding biogas digester are further based on the external data. The external data may be used to further optimize production of biogas, for example when taking logistic constraints into account. When a more suitable agro-industrial feed is not available in the vicinity of the biogas digester, it may be preferred to get a less high-yielding agro-industrial feed as this may be more cost efficient and / or having less impact on the environment. In another example, weather data may be used to determine the amount of heating needed to the biogas digester, while land availability and / or georeferenced data may be used to determine the maximum amount of throughput a biogas digester may have without violating environmental laws. The external data may also be used to simulate a suitable location for the development of a biogas digester. For example, a suitable location would be a location with a high availability of agro-industrial feed in the vicinity, a relatively predictable temperature throughout the year and provided away from a city / town.

In yet a further implementation of the method, the local controller of each biodigester is arranged to provide instructions to the operational components based on previously successfully transmitted instructions if the attempt to transmit, by the central controller to the local controller of the at least one biogas digester, the instructions for the operational components of the corresponding biogas digester, is unsuccessful. Advantageously, this may allow for the biogas digester to operate under similar conditions as it has been operating in case the transmission is unsuccessful. Such an unsuccessful transmission may be the result of an outage or a breakdown of components facilitating the transmission of the instructions.

In yet a further implementation of the method, the operational components comprise at least one of an inlet feeder, a digestion chamber control system, an agitator and a heater. By controlling the agro-industrial feedstock provided to the digestion chamber of the biogas digester, using an inlet feeder the amount and type of agro-industrial feedstock may be accurately managed, facilitating stable operation of the biogas digester. The digestion chamber control system may facilitate a constant pH of the agro-industrial feedstock provided in the biogas digester and may by itself or incorporation with a heater control the temperature inside the biogas digester. Advantageously, by controlling the amount and type of feedstock, the temperature and pH and by agitating the feedstock using a mixer, a large amount of control over biogas production may be provided, as a relatively large amount of parameters may be affected using the operational components. This may allow for the algorithmic model on the central controller to further control, or manipulate, the biogas production output more accurately.

In yet a further implementation of the method, the algorithmic model on the central controller is arranged to perform statistical analysis for correlating process parameter data, wherein the statistical analyses comprises at least one of linear and kernel-based methods, instance-based and neural methods, and ensemble methods. The algorithmic model is preferably a self-learning algorithmic model. As will be shown later in an example, using these methods a relatively accurate algorithmic model may be provided that is arranged to predict the yield of biogas of a biogas digester. A wide range of different methods are used, as the measured process parameter data may span a wide range of different types of data at different time scales, while having complex interrelated properties such as non-linear behavior.

In yet a further implementation of the method, the algorithmic model on the central controller is arranged to perform dynamical analysis for simulating the biochemical processes within a corresponding digester based on the process parameter data, wherein the dynamical analysis comprises at least one of dynamical analysis using anaerobic digestion model no. 1 and dynamical analysis using an anaerobic simulator, forming a digital twin. Anaerobic digestion model no. 1 is a mathematical model is used to simulate and optimize biogas production in a biogas digester and may be used as a digital twin. Using the mathematical model to form a digital twin may be advantageous as more insight and data may be gathered about the biogas digesters of the group of biogas digesters. An accurate digital twin may be used to cheaply and quickly scan a wide array of configurations of controllable parameters, e.g. the operational components, and uncontrollable parameters, e.g. the weather, such that the biogas production arrangement can operate in a wide arrange of situations without having to experienced them first. Preferably, the digital twin is compared to the measured process parameter data and verified. Optionally, the algorithmic model is arranged to run the digital twin in parallel to the step of determining the instructions for the operational parameters for verification and optimization purposes.

In yet a further implementation of the method, at least one biogas digester of the plurality of biogas digesters is provided at least 5 km from a second biogas digester of the plurality of biogas digesters. Providing biogas digesters at a relatively large distance from each other, e.g. at least 5 km, such as 20 km or less, or 50km or less, more data may be gathered about operating the biogas digester, which data may be processed by the algorithmic model on the central controller such that the biogas production may be optimized over a large range of external conditions, e.g. a high or low temperature. Furthermore, by spreading the biogas digesters of the biogas production arrangement, the probability of external effects, such as the weather, may be mitigated. Thus, a more continuous biogas production of the biogas production arrangement may be facilitated.

In yet a further implementation of the method, the step of manipulating the biogas production comprises at least one of selecting a agro-industrial feedstock, optimizing microbial activity and optimizing anaerobic digestion. Advantageously, the biogas production arrangement is optimized by controlling the input of the biogas digester, considering circumstances such as agro-industrial feedstock availability and expected yield, optimizing microbial activity to ensure a healthy and resilient microbial community in the biogas digester and optimizing anaerobic digestion which directly correlates to the production of biogas. Tradeoffs between these optimizations may exist, e.g. a high anaerobic digestion may not be possible due to the availability of a specific agro-industrial feedstock. The priorities of these trade-offs may be provided to the algorithmic model on the central controller, such that more weight is given to, for example, optimizing anaerobic digestion than to optimizing microbial activity if short term throughput is preferred.

A second aspect provides for a biogas digester for producing biogas. The biogas digester comprises a local controller, operational components and a sensor. The operational components are operatively connected to the local controller and arranged to manipulate biogas production in the corresponding biogas digester based on instructions provided by the local controller. The sensor is operatively connected to the local controller and arranged to measure at least one process parameter; and wherein the local controller is arranged to connect to a central controller of a biogas production arrangement. Advantageously, such a biogas digester may be operatively connected to a central controller such that it the operational components may be remotely controlled based on instructions provided by the central controller.

A third aspect provides for a data processing arrangement comprising a central controller and a data storage. The central controller is operatively connected to the central controller and arranged to perform the step of determining the instructions for operational components of at least one biogas digester as previously described. Advantageously, such a data processing arrangement may be used to determine instructions for a biogas digester, using an algorithmic model. This may simplify operating a biogas digester and optimizing its biogas yield.

A fourth aspect provides for a biogas production arrangement for optimizing production of biogas. The biogas production arrangement comprises a group of biogas digesters as previously described, and a data processing arrangement as previously described. The biogas production arrangement is arranged to carry out the steps of the method as previously described.

### BRIEF DESCRIPTION OF THE DRAWINGS

The various aspects and examples thereof will now be discussed in conjunction with drawings. In the drawings:
Figure 1: shows a schematic side view of a biogas digester; and
Figure 2: shows a schematic view of a biogas production arrangement.

### DETAILED DESCRIPTION

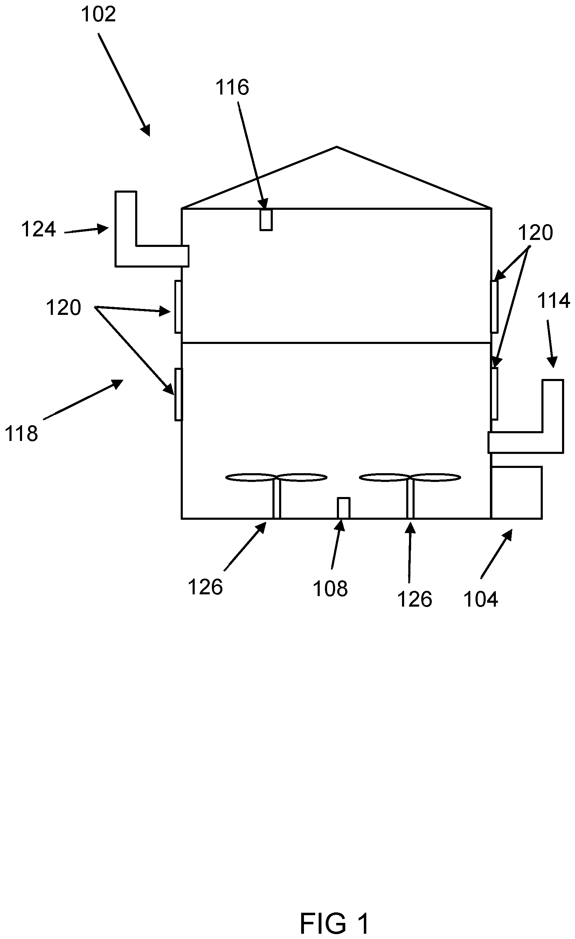

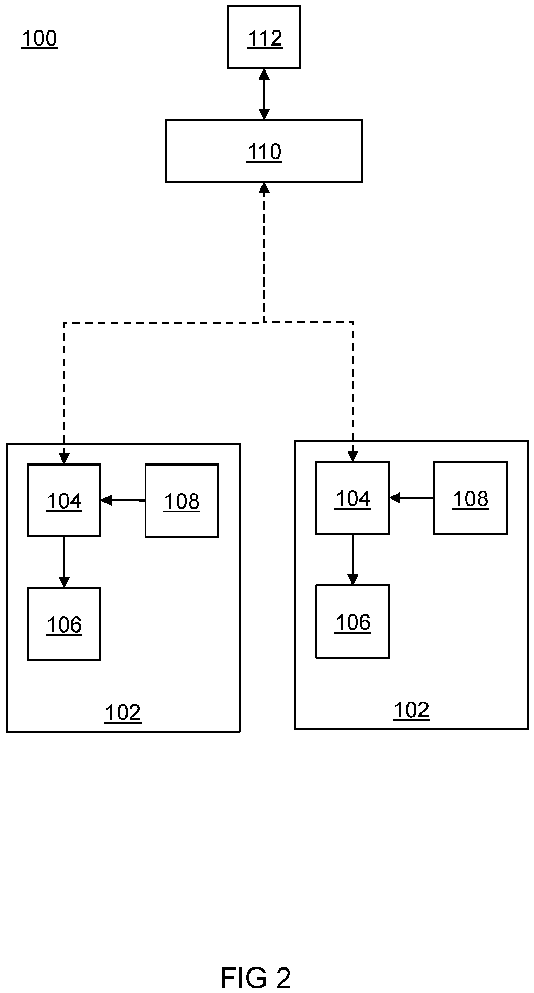

Figure 1 shows a schematic view of biogas digester 102, and Figure 2 shows an example of a biogas production arrangement 100 having a plurality of biogas digesters 102 as depicted in Figure 1. In the shown example two biogas digesters 102 have been depicted, but it will be clear to the skilled person that the group of biogas digesters 102 can comprise any number of biogas digesters 102, such as 1, 3, 4, 5 etc. The biogas digesters are provided at a distance larger than 5km from each other, in the shown example a distance of 50km is used. The biogas digester 102 is a device arranged to break down organic materials, agro-industrial feed in the shown example, in an anaerobic process. One of the break-down products is biogas, comprising methane. Other break-down products may comprise digestate, which may be as a fertilizer. Various types of biogas digesters exist, such as fixed-dome biogas digesters, floating-drum biogas digesters, plug-flow digesters and continuous stirred-tank reactors. Each type of biogas digester has its own advantages and disadvantages, and it will be clear to the skilled person that all types of biogas digesters may be used in the biogas production arrangement 100.

In the shown example, the two biogas digesters 102 are both fixed-dome biogas digesters 102. Fixed-dome biogas digesters 102 are relatively simple biogas digesters, and as a result relatively cheap to produce and maintain. As an example, the fixed-dome biogas digester 102 comprises a digestion chamber 122, or fermentation pit, in which anaerobic bacteria break down the agro-industrial feed and producing biogas.

Each biogas digester 102 comprises a local controller 104 and operational components 106 operatively connected to the local controller 104. The operational components 106 are arranged to manipulate the biogas production of the corresponding biogas digester 102. The operational components 106 of the biogas digester 102 of the example comprise various components. First, an inlet feed system 114 is provided arranged to control the amount and type of agro-industrial feed entering the digester 102. The feed may be transported from a different location, e.g. a storage, and may come from various sources in which each source comprises a different agro-industrial feed. The inlet feed system 114 may cooperate with the sensor 108, e.g. by having its own integrated measurement device, so as to measure the amount, and type of, feed provided to a corresponding biogas digester 102. This data may be a process parameter used by the algorithm on the central controller 110 to optimize biogas production.

Additionally, the operational components 106 of both biogas digesters 102 comprises a digestion chamber control system 116 arranged to control the temperature and pH of the contents of the digestion chamber 122. To control the temperature, the digestion chamber control system 116 may comprise a heating system 118. The heating system 118 of the example comprises water-jacketing 120 provided on the outside of the biogas digester 102. It will however be clear to the skilled person that the heating system may comprise additional or other heating devices, such as a internal heating coils provided in the digestion chamber 122, or an external heat exchanger, provided on the outside of the digestion chamber 122. The pH of the contents of the digestion chamber 122 may be controlled using an additive supply arrangement 124 arranged to supply an additive to the digestion chamber 122.

For example, if the pH is too high in the digestion chamber 122, e.g. above 8.0, the contents may be considered too alkaline and microbial activity may be inhibited. An acidic additive may then be provided by the additive supply arrangement 124, such as diluted sulfuric acid, to the digestion chamber 122. The introduction of the acidic additive may decrease the pH of the contents of the digestion chamber 122. If the pH of the contents in the digestion chamber 122 is too low, and the contents are too acidic, the methanogenesis of the microbes in the digestion chamber 122 is reduced and biogas production is lessened. A pH value that is too low, e.g. below 6.5, may be remedied by introduction an alkaline substance, such as calcium carbonate, using the additive supply arrangement 124. The digestion chamber control system 116 may comprise additional components to manipulate the pH value of the contents of the digestion chamber 122, e.g. ventilation arrangements, digestate recirculation configurations and mixers.

In the example, the operational components 106 further comprises a mixing system 126 provided in the digestion chamber 122 arranged to agitate and / or mix the contents of the digestion chamber 122. In the shown example, the mixing system 126 is a mechanical mixing system that is mixes the contents of the digestion chamber 122 by a rotational motion the mechanical mixers that are at least partially provided inside the content of the digestion chamber 122. Using a mixing system 126 may allow for a more homogenous distributed contents of the digestion chamber 122 and thus preventing unwanted separations of properties within the contents such as stratification. While a mechanical mixer 126 is provided, a gas recirculation system may also be provided. Such a mixer may introduce a gas in the digestion chamber 122 towards the bottom. The gas is transported through the contents of the digestion chamber 122 and is extracted towards the top of the digestion chamber 122. From here, it may be recirculated back towards the bottom of the chamber 122 where it is reintroduced in the digestion chamber 122.

Each biogas digester 102 further comprises a sensor 108 arranged to measure at least one process parameter. The local controller 104 of a biogas digester 102 is operatively connected to the sensor 108 of the corresponding biogas digester 102 such that data measured by the sensor 108 is transmitted to, and processed by, the local controller 104.

The biogas production arrangement 100 has a central controller 110 that is operatively connected to each local control 104 of the two biogas digesters 102. The central controller 110 and the data storage 112 form a data processing arrangement.

To optimise the production of biogas using the biogas production arrangement 100, the sensors 108 provided in each of the two biogas digesters 102 measure the process parameters, generating measured process parameter data. This data is processed and transmitted, e.g. using an internet connection, to the central controller 110. Using an algorithmic model provided in the central controller 110, instructions for the operational components 106 are determined. As shown in the example for the inlet feed system 114, the sensor 108 may be provided as a single sensor integrated in a component of the biogas digester 102. Additionally or alternatively, a dedicated sensor 108, or sensor system 108, may be provided to measure at least one process parameter or a plurality of process parameters.

As an example of optimizing the production of biogas, the electricity consumption is minimized over the biogas production arrangement 100. In the example, a multi-stage ensemble machine learning framework is provided as algorithmic model to improve prediction accuracy and robustness by combining multiple models. In a first step, data is gathered using the sensors 108, to measure the relevant process parameters in each of the two biogas digesters 102 and generating measured process parameter data, and by collecting data from the data storage 112. The data of the example comprises operational data, environmental data, and historical electricity consumption data.

The process parameter data of the example comprise runtime data about the corresponding bio digester 102, e.g. how long has the corresponding bio digester 102 been currently operating, the biogas production yield, biogas production schedules, and data provided by the operator regarding the biogas digester 102 individually and the biogas production arrangement 100 in total. Data provided by the operator may comprise information about abnormal behaviour of components, such as malfunctions, or unplanned interruptions of the biogas production process. The environmental data of the example comprises temperature data, humidity data and other weather-related data of the surrounding of each of the two biogas digesters 102. The historical electricity consumption data of the example comprises pricing data of electricity and seasonal trend information, which may relate the consumption of electricity of each of the bio digesters 102 to the seasonal period, e.g. a day, month, week of the year. In the example, the gathered data may be processed and used to train a plurality of machine learning models, e.g. on subsets of the gathered data.

In the example, a gradient boosting machines model is used to capture non-linear relationships between electricity consumption and operational data and / or environmental data. Furthermore, a neural network model is used to identify complex patterns and interactions of the various parameters across the operational data, environmental data and historical electricity consumption data. Additionally, a random forests model is used in the example to provide a relatively robust prediction by averaging multiple decision trees based on the data, a logistic regression model is used to capture interpretable linear relationships between the various parameters of the data, and kernel ridge regression is used to model non-linear dependencies between parameters using kernel-based techniques.

The plurality of machine learning models of the example may be ensembled in a first-level ensemble, by aggregating the outputs of the trained machine learning models into a first-level ensemble using commonly used ensemble techniques such as stacking, weighted averaging or other aggregation methods in order to combine predictions and capture complementary strengths of the individual machine learning models. Next, the first-level ensemble may be used to train a meta-learner by feeding said first-level ensemble to the meta-learner.

In the example, a meta-learner is an algorithmic model designed to learn how to learn. The meta-leaner may be used to analyse patterns across multiple learning tasks to improve for example adaptability, generalization and efficiency in new tasks, e.g. predicting electricity consumption. In the example, the meta-learner is used to refine predictions by minimizing residual errors and optimizing the combination of predictions from the first-level ensemble in the example. A prediction of the electricity consumption of the biogas production arrangement 100 may be obtained using the meta-learner from the output predictions of the meta-learner. Said prediction may be used to minimize the electricity consumption.

In the example, the results from the meta-learner the prediction accuracy of the electricity consumption may be verified. This may be done by dividing the data into training, validation and testing subsets to facilitate unbiased evaluation of the meta-leaner prediction model. The validation subset may be used to adjust hyperparameters of the parameters and optimize the performance of the machine learning models, before forming the first-level ensemble, and the meta-learner. The accuracy of the prediction of the meta-leaner may be evaluated using the test subset, wherein in the example three evaluation metrics are provided. These are the mean absolute error, used to measure the average magnitude of errors in the prediction, the mean squared error used to penalize large errors more than smaller errors, and the R-squared to determine the proportion of variance in electricity consumption predicted by the model.

By comparing the results from the meta-learner against a benchmark model, such as a single-layer machine learning model or a non-ensembled model, the effectiveness of the meta-leaner may be determined. In addition, the prediction accuracy may be monitored by comparing the predicted electricity consumption to the actual measured values the meta-leaner may be periodically retrained to improve and / or maintain the meta-leaner.

### REFERENCE NUMERALS

- 100: Biogas production arrangement
- 102: Biogas digester
- 104: Local controller
- 106: Operational components
- 108: Sensor
- 110: Central controller
- 112: Data storage
- 114: Inlet feed system
- 116: Digestion chamber control system
- 118: Heating system
- 120: Heating coils
- 122: Digestion chamber
- 124: Additive supply arrangement
- 126: Mixing system

## Claims

1. Method of producing biogas from agro-industrial feed using a biogas production arrangement, wherein the biogas production arrangement comprises:
- a group of biogas digesters, wherein each biogas digester of the group of biogas digesters comprises:
- a local controller;
- operational components operatively connected to the local controller and arranged to manipulate biogas production in the corresponding biogas digester based on instructions provided by the local controller; and
- a sensor arranged to measure at least one process parameter, said sensor operatively connected to the local controller;
wherein the biogas production arrangement further comprises:
- a central controller operatively connected to each local controller of the group of biogas digesters;
- a data storage is operatively connected to the central controller and comprising historical process parameter data;
wherein the method comprises the steps of:
- measuring the at least one process parameter of the biogas digester using each sensor of the group of biogas digesters, thereby generating measured process parameter data;
- transmitting to the central controller, by each local controller of the group of biogas digesters, the measured process parameter data;
- determining, based on the measured process parameter data and on the historical process parameter data, the instructions for the operational components of at least one biogas digester of the group of biogas digesters, using an algorithmic model provided on the central controller;
- attempt to transmit, by the central controller to the local controller of the at least one biogas digester, the instructions for the operational components of the at least one biogas digester; and, if the attempt to transmit was successful,
- instructing the operational components of the at least one biogas digester, using the local controller, such that the biogas production of the at least one biogas digester is adjusted.

2. Method of producing biogas according to claim 1, wherein the group of biogas digesters comprises a plurality of biogas digesters.

3. Method of producing biogas according to claim 2, wherein at least one biogas digester of the plurality of biogas digesters is provided at least 5 km from a second biogas digester of the plurality of biogas digesters.

4. Method of producing biogas according to any of the preceding claims, wherein the method further comprises the step of:
- appending, by the central controller, the measured process parameter data to the historical process parameter data.

5. Method of producing biogas according to any of the preceding claims, wherein the process parameter comprises at least one of:
- operational process parameter indicative of the operational performance of the corresponding biogas digester;
- microbial population process parameter indicative of the health of the microbial population in the corresponding biogas digester; and
- wherein the historical data comprises historical operational data and historical microbial population data.

6. Method of producing biogas according to claim 5, wherein the operational process parameter comprises at least one of biogas production rate data, temperature data, ammonia concentration, pH level data and microbial activity data, and wherein the operational process parameter is preferably measured in real time.

7. Method of producing biogas according to claim 5 or 6, wherein the microbial population process parameter comprises at least one of microbial function data, microbial diversity data, microbial dynamics data and microbial species data.

8. Method of producing biogas according to any of the preceding claims, wherein the data storage further comprises external data, said external data comprising at least one of weather data, georeferenced data, feedstock availability data, land data indicative of availability for digestation, and energy scarcity data, and wherein the instructions for the operational components for the at least one biogas digester are further determined on the external data.

9. Method of producing biogas according to any of the preceding claims, wherein the local controller of each biodigester is arranged to provide instructions to the operational components based on previously successfully transmitted instructions if the attempt to transmit, by the central controller to the local controller of the at least one biogas digester, the instructions for the operational components of the corresponding biogas digester, is unsuccessful.

10. Method of producing biogas according to any of the preceding claims, wherein the operational components comprise at least one of an inlet feeder, a cruncher, a pump, an agitator and a heater.

11. Method of producing biogas according to any of the preceding claims, wherein the algorithmic model on the central controller is arranged to perform statistical analysis for correlating process parameter data, wherein the statistical analyses comprises at least one of linear and kernel-based methods, instance-based and neural methods, and ensemble methods, and wherein the algorithmic model is preferably a self-learning algorithmic model.

12. Method of producing biogas according to any of the preceding claims, wherein the algorithmic model on the central controller is arranged to perform dynamical analysis for simulating the biochemical processes within a corresponding digester based on the process parameter data, wherein the dynamical analysis comprises at least one of dynamical analysis using anaerobic digestion model no. 1 and dynamical analysis using an anaerobic simulator, forming a digital twin.

13. Method of producing biogas according to any of the preceding claims, wherein the step of determining the instructions for the operational components comprises at least one of selecting a feedstock mix, optimizing microbial activity and optimizing anaerobic digestion.

14. Biogas digester for producing biogas comprising a local controller, operational components and a sensor, wherein the operational components are operatively connected to the local controller and arranged to manipulate biogas production in the corresponding biogas digester based on instructions provided by the local controller; wherein the sensor is operatively connected to the local controller and arranged to measure at least one process parameter; and wherein the local controller is arranged to connect to a central controller of a biogas production arrangement.

15. A data processing arrangement comprising a central controller and a data storage, wherein the central controller is operatively connected to the central controller and arranged to perform the step of determining the instructions for operational components of at least one biogas digester according to claim 1.

16. Biogas production arrangement for optimizing production of biogas comprising a group of biogas digesters according to claim 14, and a data processing arrangement according to claim 15, wherein the biogas production arrangement is arranged to carry out the steps of the method according to any of the preceding claims 1-13.
